(19) Europäisches Patentamt
European Patent Office
Office européen des brevets

(11) Numéro de publication : **0 354 836 B1**

(12) # FASCICULE DE BREVET EUROPEEN

(45) Date de publication du fascicule du brevet : **27.01.93 Bulletin 93/04**

(51) Int. Cl.$^5$ : **C08B 37/02,** A61K 49/04

(21) Numéro de dépôt : **89402211.0**

(22) Date de dépôt : **03.08.89**

(54) **Polymères iodés à squelette dextrane, leurs procédés de préparation et leurs applications comme produits de contraste.**

(30) Priorité : **10.08.88 FR 8810794**

(43) Date de publication de la demande : **14.02.90 Bulletin 90/07**

(45) Mention de la délivrance du brevet : **27.01.93 Bulletin 93/04**

(84) Etats contractants désignés : **AT BE CH DE ES FR GB GR IT LI LU NL SE**

(56) Documents cités :
**EP-A- 0 146 455**
**FR-A- 2 610 935**
**GB-A- 1 400 985**
**US-A- 4 406 878**

(73) Titulaire : **GUERBET S.A.**
**15, rue des Vanesses**
**F-9360 Villepinte (FR)**

(72) Inventeur : **Paris, Dominique**
**10, rue des Aulnes**
**93600 Aulnay-sous Bois (FR)**
Inventeur : **Schaefer, Michel**
**4, rue François-Girardon**
**91380 Chilly-Mazarin (FR)**
Inventeur : **Doucet, Didier**
**13, rue Amédée Dunois**
**93190 Livry-Gargan (FR)**
Inventeur : **Meyer, Dominique**
**23 rue du Tage**
**75013 Paris (FR)**

(74) Mandataire : **Le Guen, Gérard et al**
**CABINET LAVOIX 2, place d'Estienne d'Orves**
**F-75441 Paris Cédex 09 (FR)**

**EP 0 354 836 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne des polymères iodés utilisables en radiographie comme produits de contraste et notamment comme produits de contraste injectables.

Les composés utilisés comme produits de contraste injectables doivent non seulement absorber les rayons X mais également posséder diverses propriétés et notamment

- une hydrosolubilité suffisante aux pH physiologiques,
- une faible toxicité,
- une osmolarité convenable,
- une stabilité chimique dans l'organisme et à la stérilisation.

Jusqu'à présent il était nécessaire d'injecter aux patients des quantités importantes de produits de contraste pour obtenir un diagnostic correct à partir de l'image radiographique. Aussi, les recherches se sont elles orientées vers des dérivés à osmolarité plus appropriée et à toxicité réduite. Deux nouvelles catégories de produits ont ainsi fait leur apparition : les produits ioniques à faible osmolarité et les produits non ioniques.

Plus récemment on a cherché à développer une autre approche consistant à freiner la diffusion des produits opacifiants dans l'espace extra-vasculaire.

A cet effet on a proposé différents polymères iodés. C'est ainsi que US-A-3 852 341 décrit des polymères iodés obtenus par copolymérisation d'un acide 3,5-diacylamino-2,4,6-triiodobenzénique avec des composés bifonctionnels tels qu'un diépoxyde. Ces polymères sont solubles dans l'eau. FR-A-2 200 018 décrit des copolymères voisins qui sont réticulés et insolubles dans l'eau.

US-A-4 406 878 décrit un polymère iodé qui est obtenu par réaction d'anhydride tétraiodophtalique sur l'alcool polyvinylique et réticulation.

La Demanderesse a déjà mis au point des polymères iodés qui comprennent un squelette constitué par un dextrane sur lequel sont greffés des groupes présentant un motif triiodobenzénique.

Ces polymères sont obtenus notamment par conversion des groupes hydroxy du dextrane en des groupes de greffage de type carboxymethyloxy et réaction des groupes de greffage de type carboxymethyloxy ainsi obtenus avec des amines comprenant un motif triiodobenzénique.

La Demanderesse a constaté que le greffage des amines sur les groupes de greffage de type carboxy méthyloxy n'est pas complet et que de 20 à 70% de groupe de greffage de type carboxymethyloxy ne réagissent pas.

La Demanderesse a maintenant découvert qu'en faisant réagir sur les groupes de greffage de type carboxy méthyloxy ou des groupes de greffage analogues, qui subsistent après réaction avec une amine à motif triiodobenénique, une amine aliphatique, il était possible d'obtenir un polymère iodé qui présente une meilleure tolérance.

La présente invention a en conséquence pour objet des polymères iodés comprenant un squelette constitué par un dextrane sur lequel sont greffés des groupes de formule générale

$$-X-(CH_2)_n-\overset{\displaystyle O}{\overset{\|}{C}}-R \qquad (I)$$

dans laquelle
X est choisi parmi les groupes - NH-, -O- et

$$-O-\underset{\underset{\displaystyle O}{\|}}{C}-$$

n est égal à 0 à 4
et les groupes R sont simultanément

a) des groupes iodés de formule

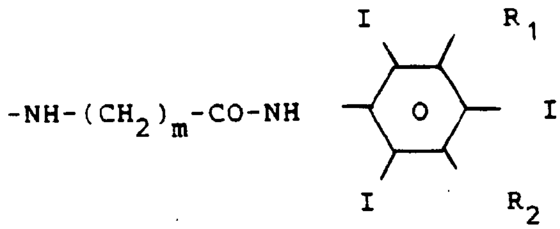

(II)

dans laquelle m est un entier de 1 à 5

$R_1$ est un groupe choisi parmi le groupe COOH, le groupe COOH salifié par une base pharmaceutiquement acceptable et les groupes de formule

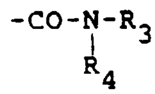

et

$R_2$ est un groupe choisi parmi les groupes de formule

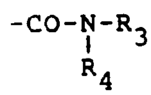

et

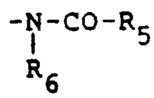

formules dans lesquelles

$R_3$ et $R_5$ sont choisis parmi les groupes alkyle en $C_1$ à $C_6$, hydroxyalkyle en $C_1$ à $C_6$, polyhydroxyalkyle en $C_1$ à $C_6$, alkoxy ($C_1$-$C_6$) alkyle ($C_1$-$C_6$) et alkoxy ($C_1$-$C_6$) hydroxyalkyle ($C_1$-$C_6$),

$R_4$ et $R_6$ sont choisis parmi un atome d'hydrogène et les groupes alkyle en $C_1$ à $C_6$, hydroxyalkyle en $C_1$ à $C_6$,polyhydroxyalkyle en $C_1$ à $C_6$, alkoxy ($C_1$-$C_6$) alkyle ($C_1$-$C_6$) et alkoxy ($C_1$-$C_6$) hydroxyalkyle ($C_1$-$C_6$),

$R_3$ pouvant être en outre un groupe de formule

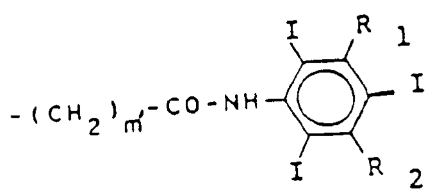

(III)

m' étant un entier de 1 à 5 et

$R_1$ et $R_2$ ayant la signification donnée précédemment.

b) des groupes non iodés de formule

-NH-R'

R' étant un groupe choisi parmi les groupes alkyle en $C_1$-$C_4$, hydroxyalkyle en $C_1$-$C_4$ et polyhydroxyalkyle ($C_1$-$C_4$).

et

c) moins de 20% de groupes hydroxy.

Le dextrane qui sert de support aux groupes triiodobenzéniques est un polymère obtenu généralement à partir de saccharose par action de Leuconostoc mesenteroïdes.

Les dextranes sont des polymères constitués par des unités d' $\alpha$ -D-glucose. Les unites glycosyle de chaînes sont liées par des enchaînements essentiellement 1 6 selon le schéma suivant

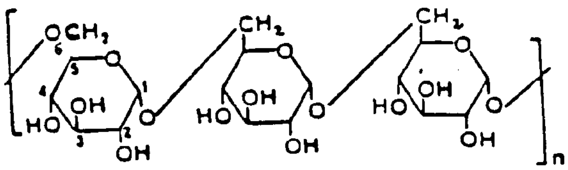

Les dextranes du commerce ont généralement subi une hydrolyse partielle suivie d'un fractionnement pour obtenir des polymères plus homogènes du point de vue de la masse moléculaire.

Les dextranes utilisés dans la présente invention peuvent avoir notamment des masses de 3.000 à 150.000 et ont de préférence des masses de 10.000 à 100.000 pour fournir des polymères iodés suffisamment hydrosolubles.

Les polymères selon l'invention peuvent être obtenus par un procédé dans lequel

a) on fait réagir sur un dextrane un réactif de greffage de façon à convertir des groupes hydroxy du dextrane en groupes de greffage de formule

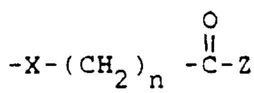 (IV)

dans laquelle X et n ont la signification donnée précédemment et Z représente un groupe d'activation.

b) on fait réagir sur le dextrane présentant des groupes de greffage ainsi obtenu une amine de formule

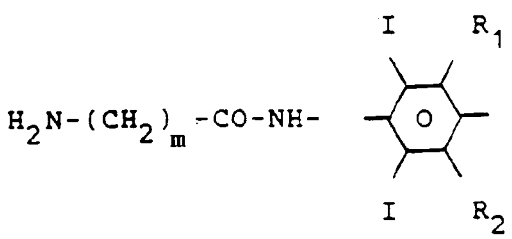 (V)

dans laquelle m, $R_1$ et $R_2$ ont la signification donnée précédemment et

c) on fait réagir sur le polymère iodé ainsi obtenu une amine de formule

$$H_2N\text{-}R' \qquad (VI)$$

R' ayant la signification donnée précédemment.

Pour la fixation de groupes de greffage on peut utiliser notamment la méthode à l'acide monochloroacétique.

Cette méthode consiste à faire réagir l'acide monochloroacétique en milieu alcalin selon le schéma réactionnel suivant

$$\vdash OH + ClCH_2COONa \xrightarrow{OH^-} \vdash OCH_2COONa + Cl^- + H_2O$$

D'une manière générale la réaction du carboxyméthyl dextrane obtenu avec une amine $RNH_2$ conduit à un dextrane sur lequel sont greffés des groupes $-OCH_2$-CO-NHR selon le schéma suivant

$$\vdash OCH_2COONa + RNH_2 \rightarrow \vdash OCH_2\text{-}CO\text{-}NH\text{-}R$$

Selon la présente on utilise successivement comme amines une amine à groupe triiodobenzénique de formule V puis une amine aliphatique de formule VI.

En pratique les réactions avec les amines nécessitent un agent de couplage. A cet effet on peut utiliser des agents de couplage classiquement utilisés pour la synthèse des peptides tels que les carbodiimides et la N-éthoxy carbonyl-2-éthoxy-1, 2-dihydroquinoléine (EEDQ). On peut également utiliser comme agent de couplage l'hexaméthyldisilazane de formule $(CH_3)_3SiNHSi(CH_3)_3$.

Pour la fixation des groupes de greffage on peut également utiliser la méthode à l'anhydride succinique.

Cette méthode consiste à faire réagir l'anhydride succinique dans un solvant polaire sur le dextrane en utilisant la 4-diméthylaminopyridine comme catalyseur d'acylation, selon le schéma réactionnel suivant

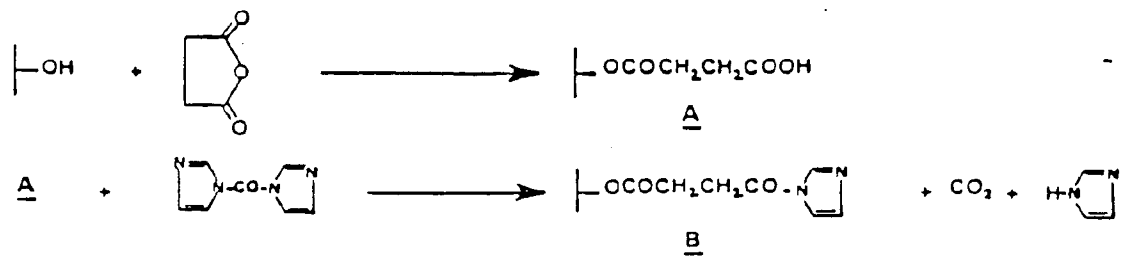

Le polymère B activé peut réagir ensuite avec des amines $RNH_2$ selon le schéma réactionnel suivant

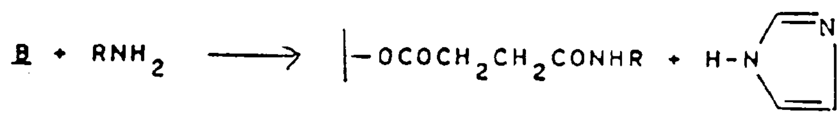

Des amines iodées de formule V sont décrites notamment dans FR-A-2272640. Comme exemple de telles amines on peut citer les amines de formules suivantes

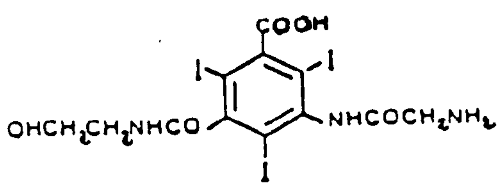

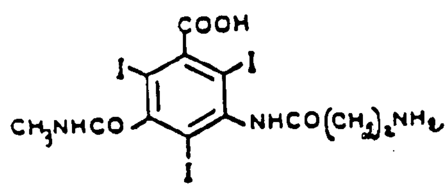

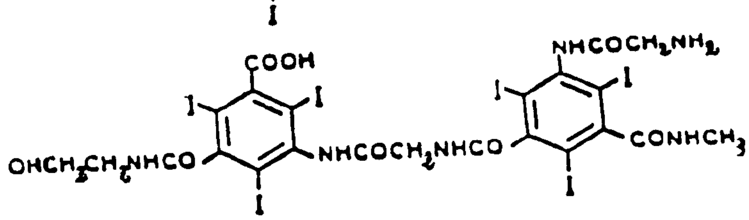

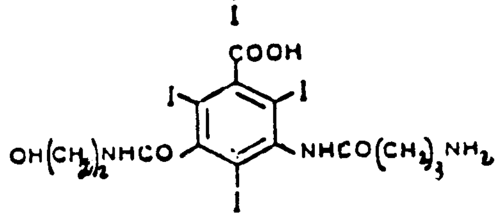

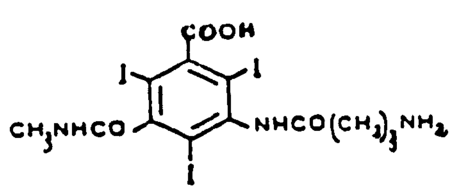

Les exemples 1 à 9 suivants illustrent les deux premiers stades de la préparation des polymères iodés. L'exemple 10 illustre le dernier stade.

Dans ces exemples les taux d'iode ont été mesurés par :

* analyse élémentaire
* argentimétrie
* spectroscopie UV

De plus, la plupart des produits ont été caractérisés par dosage acide-base dans différents solvants (eau/eau-acétone/DMSO).

Toutes les masses moléculaires ont été mesurées par chromatographie d'exclusion stérique sous haute pression, désignée également chromatographie par perméation de gel (GPC) en comparaison avec les dextranes de départ. Les masses ainsi déterminées sont désignées $M_{GPC}$ dans ce qui suit.

Exemple 1

20 de dextrane (Dextran T40, de Pharmacia Fine Chemicals, masse moléculaire moyenne en poids indiquée Mw = 40 000, $M_{GPC}$ = 27 000) sont dissous dans 165 ml d'une solution aqueuse de soude 6M à 0°C. La solution est agitée à cette température pendant 20 minutes.

41 g d'acide monochloroacétique sont ajoutés dans le milieu réactionnel. La température est alors portée à 60°C et la solution est maintenue 20 minutes à cette température sous agitation. Le mélange est ensuite refroidi puis neutralisé à pH 7,00 par addition de HCl concentré. Le produit est alors précipité dans 1 litre de méthanol, filtré, lavé et séché à 50°C sous vide.

24 g de carboxyméthyldextrane, sel de sodium sont obtenus, avec un degré de substitution de 50% (déterminé par dosage acide-base).

Cette même opération est répétée une seconde fois; 26 g de carboxyméthyldextrane, sel de sodium sont obtenus avec un degré de substitution de 80%.

La masse moléculaire du polymère obtenu, déterminé par chromatographie d'exclusion stérique est de 47 000.

2 g de carboxyméthyldextrane à 3,50 meq/g de fonctions -COONa (80% de substitution) sont dissous dans 10 ml d'eau et le pH du milieu est porté à 2,50 par addition de HCl concentré. Par ailleurs, 2,6 g de N-éthoxycarbonyl-2 éthoxy-1,2 dihydroquinoléine (EEDQ) sont dissous dans 21 ml d'éthanol et ajoutés progressivement au milieu réactionnel, sous agitation de manière homogène. Après 30 minutes, 4,6 g d'amine iodée de formule

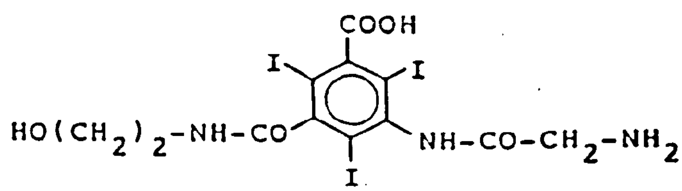

(VII)

(acide 2,4,6 triiodo-3-N-hydroxyéthylcarbamoyl-5-amino-acétamido benzoïque) dissoute dans 3,5 ml de soude 2M sont ajoutés au milieu réactionnel, le pH étant fixé à 8,50.

La réaction est laissée sous agitation à température ambiante pendant 4 heures. Puis le milieu réactionnel est évaporé sous vide pour éliminer l'éthanol avant d'être précipité dans 200 ml de méthanol. Le produit est séché sous vide à 50°C.

Le taux d'iode sur le polymère a été estimé à 6,2%.

Une seconde fixation est conduite avec les mêmes quantités de réactif pour aboutir à un taux d'iode de 16%.

Une troisième fixation avec 1 eq de EEDQ et 0,9 eq d'amine par rapport au carboxyméthyldextrane de départ permet d'amener le taux d'iode à 24%. Après avoir été séché sous vide, le polymère est redissous dans l'eau, ultrafiltré et lyophilisé.

1,5 g de carboxyméthyldextrane iodé sous forme de sel de sodium sont obtenus avec une masse moléculaire de 48000 (déterminé par GPC). Le polymère obtenu présente des groupes greffés de formule

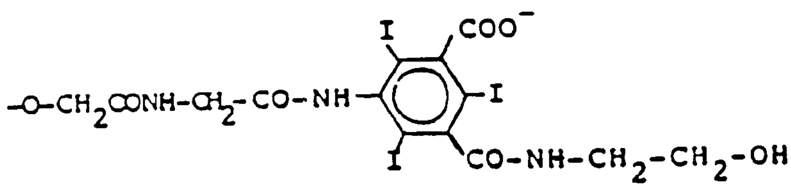

Exemple 2

1,5 g de carboxyméthyldextrane (précédemment obtenu dans l'exemple 1) à 3,50 meq/g de fonctions -COOH sous la forme acide sont dissous dans un mélange DMAC (20ml)/$H_2O$ (10 ml); 3,4 g de dicyclohexycarbodiimide dissous dans 10 ml de DMAC sont ajoutés à la solution précédente.

Quand le mélange est homogène, 3,6 g d'amine iodée de formule VII et 0,55 ml de triéthylamine dissous dans 10 ml d'eau sont ajoutés progressivement. La solution est laisée sous agitation à température ambiante pendant 24 heures, puis filtrée pour éliminer l'amine et la dicyclohéxylurée précipitées.

Le filtrat est évaporé à sec sous vide, repris à l'eau puis ultrafiltré et lyophilisé, (le sel de triéthylamine étant déplacé par de la soude).

On obtient 1,2 g de polymère, contenant 8% d'iode, avec une masse moléculaire de 48000 (déterminée par GPC).

Exemple 3

2 g de carboxyméthyldextrane (obtenu dans l'exemple 1) à 3,50 meq/g de fonctions -COONa dissous dans 5 ml d'eau et 1,5 g d'amine iodée de formule VII dissous dans 20 ml de soude 1N sont mélangés. Le pH du milieu est porté à 3,00 par addition de HCl.

Après 10 minutes, 1,6 g de chlorure de 1-éthyl-3(3-diméthylaminopropyl)-carbodiimide sont ajoutés. Le pH est maintenu constant entre 4,5 et 5,0 pendant la première heure de réaction.

La solution est laissée sous agitation à température ambiante pendant 24 heures, puis elle est filtrée. Le filtrat est ultrafiltré et lyophilisé.

On obtient 1,9 g de polymère contenant 15% d'iode, avec une masse moléculaire de 54 000 (déterminée par GPC).

Exemple 4

Le mode opératoire est identique à l'exemple 3 mais l'amine utilisée est la suivante

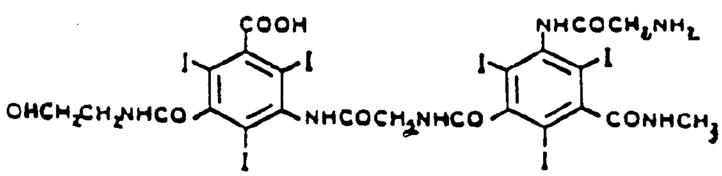

(2,9 g sont mis en réaction).

On obtient 2,3 g de polymère iodé contenant 24,6% d'iode, avec une masse moléculaire de 62 000 (déterminée par GPC).

Exemple 5

On prépare un carboxyméthyldextrane comme à l'exemple 1 mais en utilisant comme polymère de départ du Dextran T18 (Pharmacia Fine Chemicals, Mw = 18 000, $M_{GPC}$ = 14 000).

Le carboxyméthyldextrane est obtenu avec un degré de substitution de 70% et avec une masse moléculaire de 29 000 (déterminée par GPC).

2 g de carboxyméthyldextrane à 3,20 meq/g de fonctions -COONa sont dissous dans 10 ml d'eau et le pH du milieu est porté à 2,50 par addition de HCl concentré. Par ailleurs, 2,4 g de N-éthoxycarbonyl-2 éthoxy-1,2-dihydroquinoléine sont dissous dans 20 ml d'éthanol et ajoutés progressivement au milieu réactionnel, sous agitation de manière homogène.

Après 30 minutes, 5,9 g d'amine iodée de formule VII dissous dans 4,5 ml de soude 2M sont ajoutés au milieu réactionnel, le pH étant fixé à 8,50. Le mode opératoire est ensuite identique à l'exemple 1.

Le taux d'iode sur le polymère a été estimé à 6,2%.

Une seconde fixation (1,4 eq de EEDQ et 1,1 eq d'amine par rapport au carboxyméthyldextrane) per-met d'obtenir un taux d'iode de 16%, une troisième fixation (1,3 eq de EEDQ et 1,0 eq d'amine par rapport au CMD) conduit à un taux d'iode de 24% sur le polymère.

Après avoir été séché sous vide, le polymère est redissous dans l'eau, ultrafiltré et lyophilisé.

1,9 g de carboxyméthyldextrane iodé sous forme de sel de sodium sont obtenus, avec une masse moléculaire de 32 000 (déterminée par GPC).

Exemple 6

On prépare un carboxyméthyldextrane comme à l'exemple 1 mais on utilise du Dextran T10 (Pharmacia Fine Chemicals, Mw = 10 000, $M_{GPC}$ = 7 400).

Le carboxyméthyldextrane est obtenu avec un degré de substitution de 75% (3,5 meq/g) et avec une masse moléculaire de 15 000 (déterminée par GPC) alors que celle du dextrane de départ est de 7 400.

2 g de carboxyméthyldextrane à 3,35 meq/g de fonctions -COONa sont dissous dans 10 ml d'eau et le pH du milieu est porté à 2,50 par addition de HCl co-centré. Par ailleurs, 2,5 g de N-éthoxycarbonyl-2 éthoxy-1,2-dihydroquinoléine sont dissous dans 20 ml d'éthanol et ajoutés progressivement au milieu réactionnel, sous agitation de manière homogène.

Après 30 minutes, 4,6 g d'amine iodée de formule VII dissous dans 3,5 ml de soude 2M sont ajoutés au milieu réactionnel, le pH étant fixé à 8,50.

Le mode opératoire est ensuite identique à l'exemple 1.

Le taux d'iode sur le polymère est estimé à 13%.

Une seconde fixation (1,4 eq de EEDQ et 0,9 eq d'amine par rapport au carboxyméthyldextrane) permet d'obtenir un taux d'iode de 22%; une troisième fixation (1,0 eq de EEDQ et 1,0 eq d'amine par rapport au carboxyméthyldextrane) conduit à un taux d'iode de 26% sur le polymère.

Après avoir été séché sous vide, le polymère est redissous dans l'eau, ultrafiltré et lyophilisé.

1,8 g de carboxyméthyldextrane iodé sous forme de sel de sodium sont obtenus, avec une masse moléculaire de 15 000 (déterminée par GPC).

Exemple 7

0.25 g de carboxyméthyldextrane (obtenu dans l'exemple 1) à 3,50 meq/g de fonctions -COOH mises sous la forme acide sont dissous dans 10 ml de diméthylformamide, puis sont ajoutés 3 ml d'hexaméthyldisilazane. Le milieu réactionnel est porté à 100°C pendant 16 heures, puis celui-ci est évaporé et repris deux fois dans le DMF.

1,4 g d'amine iodée de formule VII sont alors ajoutés, le chauffage est porté à 50°C pendant 6 heures. Après refroidissement, le milieu réactionnel est dilué dans 100 ml d'eau. Après 1 heure, celui-ci est concentré puis précipité dans le méthanol.

Après avoir été séché sous vide, le polymère est redissous dans l'eau, ultrafiltré et lyophilisé.

On obtient 0,2 g de carboxyméthyldextrane iodé sous forme de sel de sodium avec un taux d'iode de 19,8 % et une masse moléculaire de 48 000 (déterminée par GPC).

Exemple 8

2 g de carboxyméthyldextrane à 3,20 meq/g de fonctions -COONa (obtenu à partir de dextrane de Mw 40 000) sont dissous dans 10 ml d'eau, le pH du milieu est porté à 2,50 par addition de HCl concentré. Par ailleurs, 2 équivalents de N-éthoxycarbonyl-2 éthoxy 1-2-dihydroquinoléine et 1,5 eq d'amine iodée de formule VII sont ajoutés selon le mode opératoire décrit dans l'exemple 1.

Une deuxième fixation est conduite avec 1,5 eq d'EEDQ et 1,5 eq d'amine par rapport au carboxyméthyldextrane de départ.

On obtient 1,5 g de polymère iodé contenant 35% d'iode, avec une masse moléculaire de 50 000.

Exemple 9

3 g de dextrane (Mw = 40 000, MGPC = 27 000) sont dissous dans 30 ml de DMSO, 7,2 g d'anhydride succinique dans 35 ml de DMSO sont ajoutés à la solution précédente ainsi que 1,8 g de 4-diméthylaminopyridine sous forme solide. La solution est agitée pendant 4 heures à 45°C. Le milieu réactionnel est précipité dans du méthanol. Après séchage, il est redissous dans de l'eau puis ultrafiltré et lyophilisé. 3 g de polymère sont obtenus avec un taux de substitution de 131%.

1 g du polymère précédemment obtenu à 4,87 meq/g de fonctions acides est dissous dans 20 ml de DMSO avec 1,6 g de carbonyldiimidazole.

La solution est agitée à température ambiante pendant 30 minutes. Puis 6 g d'amine iodée de formule VII dissous dans 4,5 ml de soude 2M sont ajoutés; l'agitation est laissée pendant 24 heures. Le milieu réactionnel est précipité dans du méthanol. Après séchage, le produit est redissous dans de l'eau, ultrafiltré et lyophilisé.

Le polymère obtenu possède un taux d'iode de 20%.

Le polymère obtenu présente des groupes greffés de formule

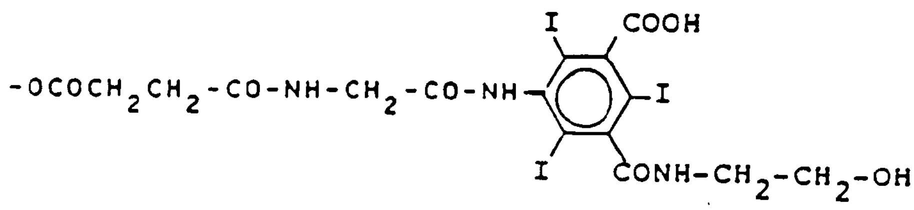

Exemple 10

10 g de carboxymethyldextrane iodé préparé comme décrit à l'Exemple 1, (ayant un taux d'iode de 25,5%, $M_w$ = 65400, Mn = 33000 ; indice de polydispersité = 2,0) sont dissous dans 30 ml d'eau. Le pH du milieu est porté à 3,50 par addition de HCl concentré. Par ailleurs, 5,2 g (0,019 mole) de N-éthoxycarbonyl-2 éthoxy-1,2 dihydroquinoléine sont dissous dans 30 ml d'éthanol et ajoutés progressivement au milieu réactionnel, sous agitation de manière homogène.

Après 30 minutes, 1,2 g d'éthanolamine (0,020 mole) sont ajoutés au milieu réactionnel.

La réaction est laissée sous agitation à température ambiante pendant 24 heures. Puis, le milieu réactionnel est évaporé sous vide pour éliminer l'éthanol avant d'être précipité dans 500 ml de méthanol.

Le produit est séché sous vide à 50°C.

Une seconde fixation d'éthanolamine est conduite dans les mêmes conditions, avec les mêmes quantités de réactifs.

Après avoir été séché sous vide, le polymère est redissous dans l'eau, ultrafiltré; la solution est évaporée. Le produit obtenu est repris à l'éther puis séché sous vide à 50°C.

7,5 g de carboxyméthyldextrane iodé modifié éthanolamine sont obtenus avec

$M_W$ = 47500
$M_n$ = 25300
Indice de polydispersité = 1,9

Le polymère obtenu présente

. 54% de groupes greffés de formule

$$-OCH_2CONHCH_2CH_2OH$$

. 30% de groupes greffés de formule

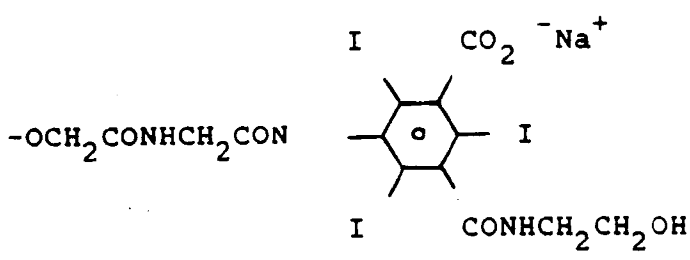

(% iode = 26,6)

. 16 % de groupes carboxymeth yliques non modifiés.

| | | |
|---|---|---|
| solubilité % m/v | : | 33,7 soit 8,96% iode |
| Osmolarité mOsm/Kg (20°C) | : | 440 (cryoscopie) |
| | : | 875 (tonométrie) |
| Viscosité mPa/s (37°C) | : | 26 |

Exemple 11

On a opéré comme à l'exemple 10, mais en utilisant à la place de l'éthanolamine de la méthylamine.

Le polymère iodé obtenu a les caractéristiques suivantes :

$M_w$ = 23000
$M_n$ = 11000
Indice de polydispersite : 2,1
Concentration en iode : 24, 1 %.

Exemple 12

On a opéré comme à l'exemple 10, mais en utilisant à la place de l'éthanolamine de la 2,3-dihydroxypropylamine.

Le polymère iodé a les caractéristiques suivantes :

$M_w$ = 21500
$M_n$ = 12000
Indice de polydispersité : 1,8
Concentration en iode : 20 %.

On donnera ci-après des résultats comparés relatifs à la toxicité des polyméres avant réaction et après réaction avec une amine aliphatique.

| Polymère | Exemple 1 | Exemple 10 |
|---|---|---|
| type | avant réaction | après réaction |
| DL50, IV souris 2 ml/min (ml/Kg) | 12,9 | > 50 |
| (mEq motif glucose/Kg) | 9 | > 39,1 |

Ces résultats mettent en évidence une toxicité plus de 3 fois plus faible pour le polymère de l'exemple 10 obtenu après réaction d'un polymère iodé avec une amine aliphatique.

La présente invention a également pour objet des produits de contraste qui comprennent au moins un polymère iodé tel que défini précédemment.

Ces produits de contraste sont utilisables chez l'homme et les animaux à des fins radiologiques.

La forme pharmaceutique préférée des produits de contraste selon l'invention est constituée par des solutions aqueuses des polymères.

On donnera ci-après un exemple de produit de contraste selon la présente invention

| | |
|---|---|
| Produit de contraste | |
| Polymère de l'exemple 10 | 20 g |
| Eau pour préparation injectable | |
| Q.S.P. | 100 ml. |

D'une manière générale les produits de contraste selon l'invention peuvent être administrés par toutes les voies classiquement utilisées pour les produits de contraste et en particulier par voie parentérale (voie interveineuse, intra-artérielle, intra ou péri-lymphatique, voie sous-arachnoïdienne (myélographie)) et par voie orale.

Les produits de contraste selon la présente invention peuvent être utilisés en particulier en angiographie. On peut généralement les injecter chez l'homme à des doses d'iodes de 30 à 500 mgJ/kg.

## Revendications

### Revendications pour les Etats contractants suivants : AT, BE, CH, DE, FR, GB, GR, IT, LI, LU, NL, SE

1. Polymères iodés comprenant un squelette constitué par un dextrane sur lequel sont greffés des groupes de formule générale

$$-X-(CH_2)_n-\overset{\overset{O}{\|}}{C}-R \qquad (I)$$

dans laquelle
X est choisi parmi les groupes - NH-, -O- et

$$-O-\underset{\underset{O}{\|}}{C}-$$

n est égal à 0 à 4
et les groupes R sont simultanément
a) des groupes iodés de formule

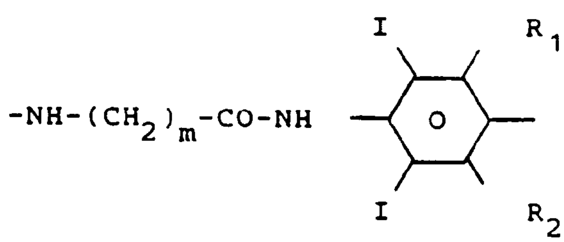 (II)

dans laquelle m est un entier de 1 à 5
$R_1$ est un groupe choisi parmi le groupe COOH, le groupe COOH salifié par une base pharmaceutiquement acceptable et les groupes de formule

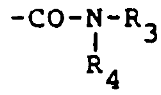

et

$R_2$ est un groupe choisi parmi les groupes de formule

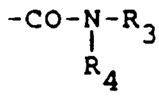

et

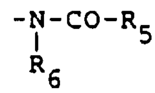

formules dans lesquelles

$R_3$ et $R_5$ sont choisis parmi les groupes alkyle en $C_1$ à $C_6$, hydroxyalkyle en $C_1$ à $C_6$, polyhydroxyalkyle en $C_1$ à $C_6$, alkoxy ($C_1$-$C_6$) alkyle ($C_1$-$C_6$) et alkoxy ($C_1$-$C_6$) hydroxyalkyle ($C_1$-$C_6$),

$R_4$ et $R_6$ sont choisis parmi un atome d'hydrogène et les groupes alkyle en $C_1$ à $C_6$, hydroxyalkyle en $C_1$ à $C_6$, polyhydroxyalkyle en $C_1$ à $C_6$, alkoxy ($C_1$-$C_6$) alkyle ($C_1$-$C_6$) et alkoxy ($C_1$-$C_6$) hydroxyalkyle ($C_1$-$C_6$),

$R_3$ pouvant être en outre un groupe de formule

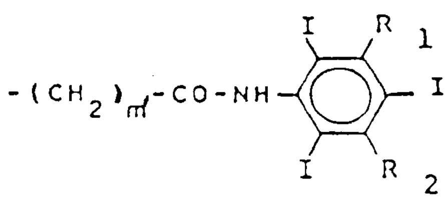

(III)

m' étant un entier de 1 à 5 et

$R_1$ et $R_2$ ayant la signification donnée précédemment.

b) des groupes non iodés de formule

-NH-R'

R' étant un groupe choisi parmi les groupes alkyle en $C_1$-$C_4$, hydroxyalkyle en $C_1$-$C_4$ et polyhydroxyalkyle ($C_1$-$C_4$).

et

c) moins de 20% de groupes hydroxy.

2. Polymères selon la revendication 1, dans lesquels les groupes greffés sont du type

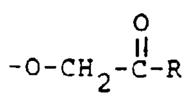

3. Procédé de préparation d'un polymère selon la revendication 1, caractérisé en ce que

a) on fait réagir sur un dextrane un réactif de greffage de façon à convertir des groupes hydroxy du dextrane en groupes de greffage de formule

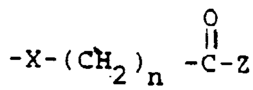 (IV)

dans laquelle X et n ont la signification donnée précédemment et Z représente un groupe d'activation.

b) on fait réagir sur le dextrane présentant des groupes de greffage ainsi obtenu une amine de formule

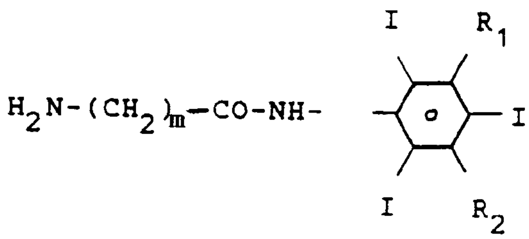 (V)

dans laquelle m, $R_1$ et $R_2$ ont la signification donnée à la revendication 1 et

c) on fait réagir sur le polymère iodé ainsi obtenu une amine de formule

$H_2N\text{-}R'$ (VI)

R' ayant la signification donnée précédemment.

4. Produit de contraste pour la radiographie, caractérisé en ce qu'il contient un polymère selon l'une quelconque des revendications 1 et 2.

5. Produit de contraste selon la revendication 4, caractérisé en ce qu'il est constitué par une solution aqueuse du polymère.

**Revendications pour l'Etat contractant suivant : ES**

1. Procédé de préparation de polymères iodés comprenant un squelette constitué par un dextrane sur lequel sont greffés des groupes de formule générale

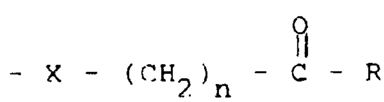 (I)

dans laquelle

X est choisi parmi les groupes - NH-, -O- et

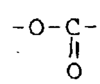

n est égal à 0 à 4

et les groupes R sont simultanément

a) des groupes iodés de formule

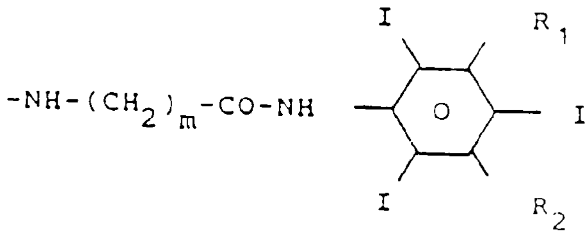

(II)

dans laquelle m est un entier de 1 à 5

$R_1$ est un groupe choisi parmi le groupe COOH, le groupe COOH salifié par une base pharmaceutiquement acceptable et les groupes de formule

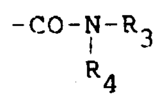

et

$R_2$ est un groupe choisi parmi les groupes de formule

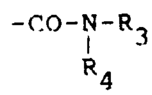

et

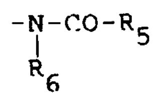

formules dans lesquelles

$R_3$ et $R_5$ sont choisis parmi les groupes alkyle en $C_1$ à $C_6$, hydroxyalkyle en $C_1$ à $C_6$, polyhydroxyalkyle en $C_1$ à $C_6$, alkoxy ($C_1$-$C_6$) alkyle ($C_1$-$C_6$) et alkoxy ($C_1$-$C_6$) hydroxyalkyle ($C_1$-$C_6$),

$R_4$ et $R_6$ sont choisis parmi un atome d'hydrogène et les groupes alkyle en $C_1$ à $C_6$, hydroxyalkyle en $C_1$ à $C_6$, polyhydroxyalkyle en $C_1$ à $C_6$, alkoxy ($C_1$-$C_6$) alkyle ($C_1$-$C_6$) et alkoxy ($C_1$-$C_6$) hydroxyalkyle ($C_1$-$C_6$),

$R_3$ pouvant être en outre un groupe de formule

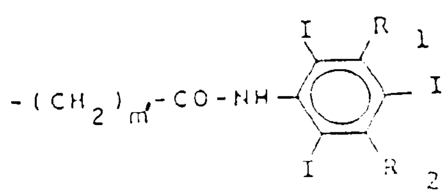

(III)

m' étant un entier de 1 à 5 et

$R_1$ et $R_2$ ayant la signification donnée précédemment.

b) des groupes non iodés de formule

$$-NH-R'$$

R' étant un groupe choisi parmi les groupes alkyle en $C_1$-$C_4$, hydroxyalkyle en $C_1$-$C_4$ et polyhydroxyalkyle ($C_1$-$C_4$).

et

c) moins de 20% de groupes hydroxy, caractérisé en ce que :

a) on fait réagir sur un dextrane un réactif de greffage de façon à convertir des groupes hydroxy du dextrane en groupes de greffage de formule

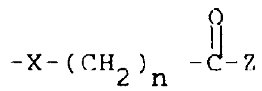

(IV)

dans laquelle X et n ont la signification donnée précédemment et Z représente un groupe d'activation,

b) on fait réagir sur le dextrane présentant des groupes de greffage ainsi obtenu une amine de formule

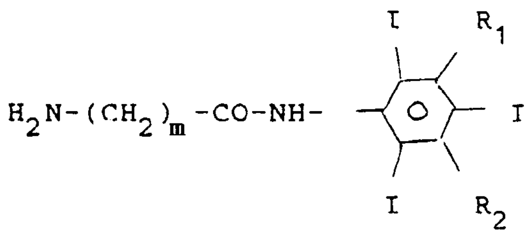

(V)

dans laquelle m, $R_1$ et $R_2$ ont la signification donnée précédemment et

c) on fait réagir sur le polymère iodé ainsi obtenu une amine de formule

$H_2N\text{-}R'$ (VI)

R' ayant la signification donnée précédemment.

2. Procédé de préparation d'un produit de contraste pour la radiographie, caractérisé en ce qu'il consiste à mettre un polymère obtenu par un procédé selon la revendication 1 sous une forme administrable.

**Patentansprüche**

**Patentansprüche für folgende Vertragsstaaten : AT, BE, CH, DE, FR, GB, GR, IT, LI, LU, NL, SE**

1. Iodhaltige Polymere mit einem Dextrangerüst, auf das Gruppen der allgemeinen Formel

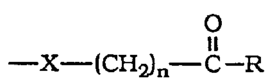

(I)

aufgepfropft sind, in der
X ausgewählt ist aus Gruppen -NH-, -O- und

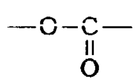

n 0 bis 4 bedeutet und
die Gruppen R gleichzeitig

a) iodhaltige Gruppen der Formel

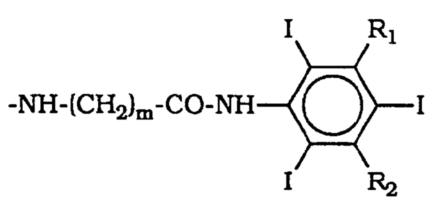

(II)

in der m eine ganze Zahl mit einem Wert von 1 bis 5,

$R_1$ eine Gruppe, ausgewählt aus Gruppen COOH, Gruppen COOH, die mit einer pharmazeutisch annehmbaren Base in ein Salz überführt worden sind, und Gruppen der Formel

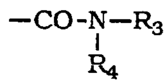

und
$R_2$ eine Gruppe ausgewählt aus Gruppen der Formel

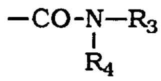 und 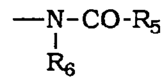

darstellen, worin
$R_3$ und $R_5$ ausgewählt sind aus $C_1$-$C_6$-Alkylgruppen, $C_1$-$C_6$-Hydroxyalkylgruppen, $C_1$-$C_6$-Polyhydroxyalkylgruppen, $C_1$-$C_6$-Alkoxy-$C_1$-$C_6$-alkylgruppen und $C_1$-$C_6$-Alkoxy-$C_1$-$C_6$-hydroxyalkylgruppen,
$R_4$ und $R_6$ ausgewählt sind aus Wasserstoffatomen und $C_1$-$C_6$-Alkylgruppen, $C_1$-$C_6$-Hydroxyalkylgruppen, $C_1$-$C_6$-Polyhydroxyalkylgruppen, $C_1$-$C_6$-Alkoxy-$C_1$-$C_6$-alkylgruppen und $C_1$-$C_6$-Alkoxy-$C_1$-$C_6$-hydroxyalkylgruppen,
wobei $R_3$ zusätzlich eine Gruppe der Formel

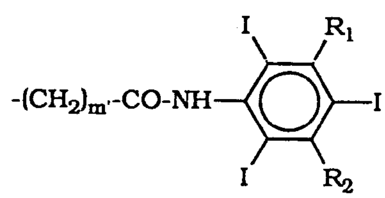 (III)

darstellen kann,
worin m' eine ganze Zahl mit einem Wert von 1 bis 5 darstellt und
$R_1$ und $R_2$ die oben angegebenen Bedeutungen besitzen,
b) nicht-iodhaltige Gruppen der Formel

-NH-R'

worin R' eine Gruppe ausgewählt aus $C_1$-$C_4$-Alkylgruppen, $C_1$-$C_4$-Hydroxyalkylgruppen und $C_1$-$C_4$-Polyhydroxyalkylgruppen darstellt, und
c) weniger als 20 % Hydroxylgruppen bedeuten.

**2.** Polymere nach Anspruch 1, worin die aufgepfropften Gruppen der Formel

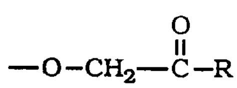

entsprechen.

**3.** Verfahren zur Herstellung eines Polymers nach Anspruch 1, **dadurch gekennzeichnet**, daß man
a) auf ein Dextran ein Pfropfreagens einwirken läßt, um die Hydroxylgruppen des Dextrans in Pfropfgruppen der Formel

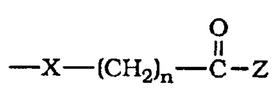 (IV)

umzuwandeln, worin X und n die oben angegebenen Bedeutungen besitzen und Z eine Aktivierungsgruppe darstellt,
b) auf das in dieser Weise erhaltene Dextran mit Pfropfgruppen ein Amin der Formel

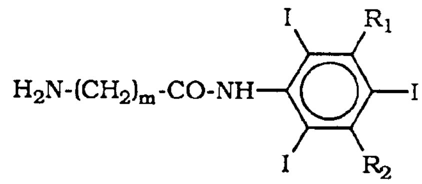 (V)

einwirken läßt, in der m, $R_1$ und $R_2$ die in Anspruch 1 angegebenen Bedeutungen besitzen, und
c) auf das in dieser Weise erhaltene iodhaltige Polymer ein Amin der Formel

(VI) $H_2N - R'$

einwirken läßt, in der R′ die oben angegebenen Bedeutungen besitzt.

4. Röntgenkontrastmittel, **dadurch gekennzeichnet**, daß es ein Polymer nach einem der Ansprüche 1 und 2 enthält.

5. Röntgenkontrastmittel nach Anspruch 4, **dadurch gekennzeichnet**, daß es eine wäßrige Lösung des Polymers umfaßt.

**Patentansprüche für folgenden Vertragsstaat : ES**

1. Verfahren zur Herstellung von iodhaltigen Polymeren mit einem Dextrangerüst, auf das Gruppen der allgemeinen Formel

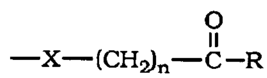 (I)

aufgepfropft sind, in der
X ausgewählt ist aus Gruppen -NH-, -O- und

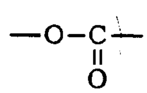

n 0 bis 4 bedeutet und
die Gruppen R gleichzeitig
a) iodhaltige Gruppen der Formel

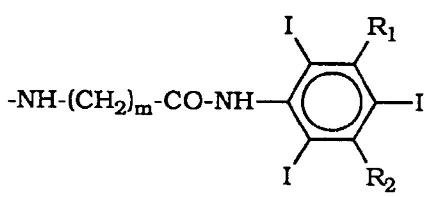 (II)

in der m eine ganze Zahl mit einem Wert von 1 bis 5,
$R_1$ eine Gruppe, ausgewählt aus Gruppen COOH, Gruppen COOH, die mit einer pharmazeutisch annehmbaren Base in ein Salz überführt worden sind, und Gruppen der Formel

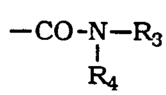

und
$R_2$ eine Gruppe ausgewählt aus Gruppen der Formel

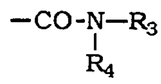 und 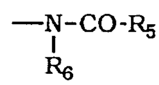

darstellen, worin
$R_3$ und $R_5$ ausgewählt sind aus $C_1$-$C_6$-Alkylgruppen, $C_1$-$C_6$-Hydroxyalkylgruppen, $C_1$-$C_6$-Polyhydroxyalkylgruppen, $C_1$-$C_6$-Alkoxy-$C_1$-$C_6$-alkylgruppen und $C_1$-$C_6$-Alkoxy-$C_1$-$C_6$-hydroxyalkylgruppen,
$R_4$ und $R_6$ ausgewählt sind aus Wasserstoffatomen und $C_1$-$C_6$-Alkylgruppen, $C_1$-$C_6$-Hydroxyalkylgruppen, $C_1$-$C_6$-Polyhydroxyalkylgruppen, $C_1$-$C_6$-Alkoxy-$C_1$-$C_6$-alkylgruppen und $C_1$-$C_6$-Alkoxy-$C_1$-$C_6$-hydroxyalkylgruppen,
wobei $R_3$ zusätzlich eine Gruppe der Formel

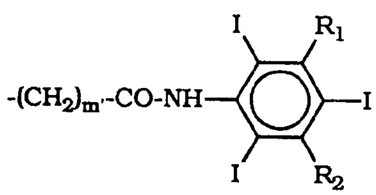 (III)

darstellen kann,
worin m′ eine ganze Zahl mit einem Wert von 1 bis 5 darstellt und $R_1$ und $R_2$ die oben angegebenen Bedeutungen besitzen,
b) nicht-iodhaltige Gruppen der Formel

-NH-R′

worin R′ eine Gruppe ausgewählt aus $C_1$-$C_4$-Alkylgruppen, $C_1$-$C_4$-Hydroxyalkylgruppen und $C_1$-$C_4$-Polyhydroxyalkylgruppen darstellt, und
c) weniger als 20 % Hydroxylgruppen bedeuten,
**dadurch gekennzeichnet**, daß man
a) auf ein Dextran ein Pfropfreagens einwirken läßt, um die Hydroxylgruppen des Dextrans in Pfropfgruppen der Formel

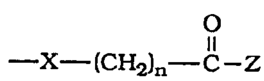 (IV)

umzuwandeln, worin X und n die oben angegebenen Bedeutungen besitzen und Z eine Aktivierungsgruppe darstellt,
b) auf das in dieser Weise erhaltene Dextran mit Pfropfgruppen ein Amin der Formel

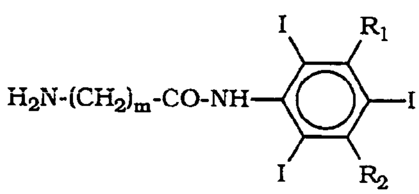 (V)

einwirken läßt, in der m, $R_1$ und $R_2$ die in Anspruch 1 angegebenen Bedeutungen besitzen, und
c) auf das in dieser Weise erhaltene iodhaltige Polymer ein Amin der Formel

$$H_2N - R' \quad (VI)$$

einwirken läßt, in der R′ die oben angegebenen Bedeutungen besitzt.

2. Verfahren zur Herstellung eines Röntgenkontrastmittels, **dadurch gekennzeichnet**, daß man ein nach dem Verfahren nach Anspruch 1 erhaltenes Polymer in eine verabreichbare Form überführt.

## Claims

### Claims for the following Contracting States : AT, BE, CH, DE, FR, GB, GR, IT, LI, LU, NL, SE

1. Iodinated polymers comprising a skeleton constituted by a dextran on which are grafted groups of general formula:

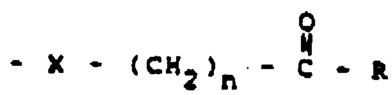 (I)

in which
X is selected from among the groups -NH-, -O- and

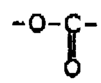

n is equal to 0 to 4
and the R groups are simultaneously
a) iodinated groups of formula

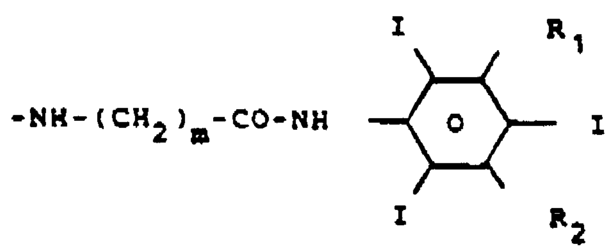 (II)

in which m is an integer of 1 to 5
$R_1$ is a group selected from among the COOH group, the COOH group made into a salt by a pharmaceutically acceptable base and the groups of formula

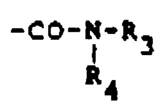

and
$R_2$ is a group selected from among the groups of formula

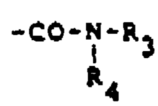

and

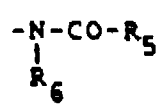

formulae in which
$R_3$ and $R_5$ are selected from among $C_1$ to $C_6$ alkyl, $C_1$ to $C_6$ hydroxyalkyl, $C_1$ to $C_6$ polyhydroxyalkyl,

($C_1$-$C_6$) alkoxy ($C_1$-$C_6$) alkyl and ($C_1$-$C_6$) alkoxy ($C_1$-$C_6$) hydroxyalkyl groups,
$R_4$ and $R_6$ are selected from among a hydrogen atom and $C_1$ to $C_6$ alkyl, $C_1$ to $C_6$ hydroxyalkyl, $C_1$ to $C_6$ polyhydroxyalkyl, ($C_1$-$C_6$) alkoxy ($C_1$-$C_6$) alkyl and ($C_1$-$C_6$) alkoxy ($C_1$-$C_6$) hydroxyalkyl groups,
$R_3$ may be, in addition, a group of formula

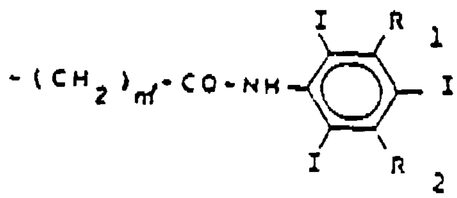 (III)

m′ being an integer of 1 to 5 and
$R_1$ and $R_2$ having the meaning given previously
b) non-iodinated groups of formula

-NH-R′

R′ being a group selected from among the $C_1$-$C_4$ alkyl, $C_1$-$C_4$ hydroxyalkyl and ($C_1$-$C_4$) polyhydroxyalkyl groups.
and
c) less than 208 of hydroxy groups.

2. Polymers according to claim 1, in which the grafted groups are of the type

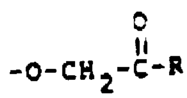

3. Process for the preparation of a polymer according to claim 1, characterized in that
a) a grafting reagent is made to react on a dextran so as to convert hydroxy groups of the dextran into grafting groups of formula

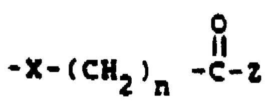 (IV)

in which X and n have the meaning given previously and Z represents an activation group.
b) on the dextran thus obtained exhibiting grafting groups, there is made to react an amine of formula

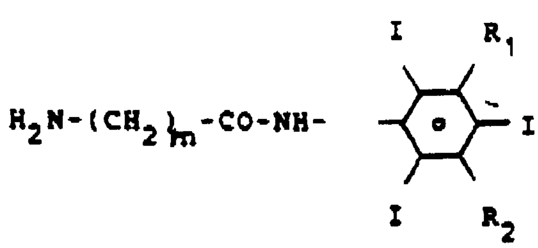 (V)

in which m, $R_1$ and $R_2$ have the meaning given in claim 1 and
c) on the iodinated polymer thus obtained, there is made to react an amine of formula

$H_2N$-R′ (VI)

R′ having the meaning given previously.

4. Contrast product for radiography, characterized in that it contains a polymer according to either one of claims 1 and 2.

5. Contrast product according to claim 4, characterized in that it is constituted by an aqueous solution of the polymer.

**Claims for the following Contracting State : ES**

1. Process for the preparation of iodinated polymers comprising a skeleton constituted by a dextran on which are grafted groups of general formula:

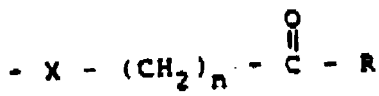 (I)

in which
X is selected from among the groups -NH-, -O- and

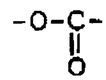

n is equal to 0 to 4
and the R groups are simultaneously
a) iodinated groups of formula

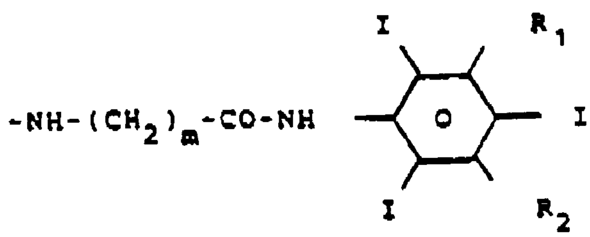 (II)

in which m is an integer of 1 to 5
$R_1$ is a group selected from among the COOH group, the COOH group made into a salt by a pharmaceutically acceptable base and the groups of formula

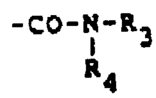

and
$R_2$ is a group selected from among the groups of formula

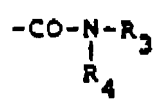

and

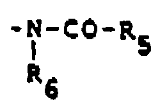

formulae in which

$R_3$ and $R_5$ are selected from among $C_1$ to $C_6$ alkyl, $C_1$ to $C_6$ hydroxyalkyl, $C_1$ to $C_6$ polyhydroxyalkyl, ($C_1$-$C_6$) alkoxy ($C_1$-$C_6$) alkyl and ($C_1$-$C_6$) alkoxy ($C_1$-$C_6$) hydroxyalkyl groups,
$R_4$ and $R_6$ are selected from among a hydrogen atom and $C_1$ to $C_6$ alkyl, $C_1$ to $C_6$ hydroxyalkyl, $C_1$ to $C_6$ polyhydroxyalkyl, ($C_1$-$C_6$) alkoxy ($C_1$-$C_6$) alkyl and ($C_1$-$C_6$) alkoxy ($C_1$-$C_6$) hydroxyalkyl groups,
$R_3$ may be, in addition, a group of formula

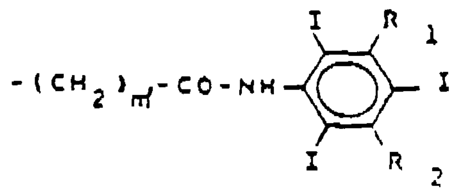

(III)

m' being an integer of 1 to 5 and
$R_1$ and $R_2$ having the meaning given previously
b) non-iodinated groups of formula

-NH-R'

R' being a group selected from among the $C_1$-$C_4$ alkyl, $C_1$-$C_4$ hydroxyalkyl and ($C_1$-$C_4$) polyhydroxyalkyl groups,
and
c) less than 20% of hydroxy groups, characterized in that:

a) a grafting reagent is made to react on a dextran so as to convert hydroxy groups of the dextran into grafting groups of formula

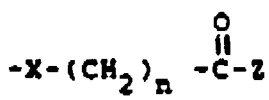

(IV)

in which X and n have the meaning given previously and Z represents an activation group,
b) on the dextran thus obtained exhibiting grafting groups, there is made to react an amine of formula

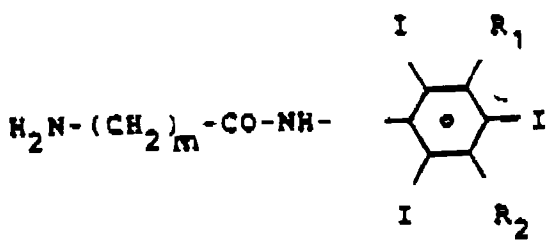

(V)

in which m, $R_1$ and $R_2$ have the meaning given previously and
c) on the iodinated polymer thus obtained, there is made to react an amine of formula

$H_2N$-R' (VI)

R' having the meaning given previously.

2. Process for the preparation of a contrast product for radiography, characterized in that it consists of placing a polymer obtained by a process according to claim 1 in an administrable form.